# EUROPEAN PATENT SPECIFICATION

(11) **EP 4 366 750 B1**
(45) Date of publication and mention of the grant of the patent: **06.05.2026**
(21) Application number: 22738002.9
(22) Date of filing: 17.06.2022
(51) Int. Cl.: A61K 35/747, G01N 33/50, A61K 35/00, A61P 11/00, A23L 33/135, C12R 1/25, C12R 1/24, C12R 1/225, C12R 1/01, C12N 1/20, C12R 1/46, C12R 1/23

(54) **SPECIES, STRAINS AND COMPOSITIONS OF LACTIC ACID BACTERIA CAPABLE OF MODULATING BODY OXYGENATION BY INCREASING HIF-1ALPHA LEVELS**
SPEZIES, STÄMME UND ZUSAMMENSETZUNGEN VON MILCHSÄUREBAKTERIEN ZUR MODULIERUNG DER KÖRPEROXYGENIERUNG DURCH ERHÖHUNG DES HIF-1-ALPHA-SPIEGELS
ESPÈCES, SOUCHES ET COMPOSITIONS DE BACTÉRIES D'ACIDE LACTIQUE APTES À MODULER L'OXYGÉNATION DU CORPS PAR AUGMENTATION DES TAUX DE HIF-1ALPHA

(30) Priority: 09.07.2021 IT 202100018152
(43) Date of publication of application: 15.05.2024
(73) Proprietor: De Simone, Claudio, 1660 Château d'Oex (CH)
(72) Inventor: De Simone, Claudio, 1660 Château d'Oex (CH)
(74) Representative: Cavattoni, Raimondi & Luppi Srl
(86) International application number: PCT/IB2022/055644
(87) International publication number: WO 2023/281335

(56) References cited:
- EP-A1- 2 455 092
- WO-A1-2015/172191
- WO-A1-2017/083470
- CN-A- 108 576 822
- CN-A- 111 084 386
- CN-A- 111 840 335
- US-A1- 2017 232 047
- US-A1- 2021 076 649
- DESANTIS SALVATORE ET AL: "Modulation of Morphology and Glycan Composition of Mucins in Farmed Guinea Fowl (Numida meleagris) Intestine by the Multi-Strain Probiotic Slab51", vol. 11, no. 2, 1 January 2021 (2021-01-01) - 1 January 2021 (2021-01-01), pages 495, XP055903460, Retrieved from the Internet <URL:https://www.ncbi.nlm.nih.gov/pmc/articles/PMC7918860/pdf/animals-11-00495.pdf> DOI: 10.3390/ani11020495
- SPAGNOLELLO ORNELLA ET AL: "Targeting Microbiome: An Alternative Strategy for Fighting SARS-CoV-2 Infection", vol. 66, no. 1-2, 1 January 2021 (2021-01-01), CH, pages 24 - 32, XP055816387, ISSN: 0009-3157, Retrieved from the Internet <URL:https://www.karger.com/Article/Pdf/515344> DOI: 10.1159/000515344
- ROSTYSLAVV BUBNOV ET AL: "Specific properties of probiotic strains: relevance and benefits for the host", THE EPMA JOURNAL, SPRINGER, NL, vol. 9, no. 2, 13 April 2018 (2018-04-13), pages 205 - 223, XP021256829, ISSN: 1878-5077, DOI: 10.1007/S13167-018-0132-Z
- WING PETER A.C. ET AL: "Hypoxic and pharmacological activation of HIF inhibits SARS-CoV-2 infection of lung epithelial cells", 5 April 2021 (2021-04-05), pages 1 - 13, XP055821018, Retrieved from the Internet <URL:https://www.ncbi.nlm.nih.gov/pmc/articles/PMC8020087/?report=printable> [retrieved on 20210705], DOI: 10.1016/j.celrep.2021.109020
- SEREBROVSKA ZOYA O ET AL: "Hypoxia, HIF-1[alpha], and COVID-19: from pathogenic factors to potential therapeutic targets", ACTA PHARMACOLOGICA SINICA, vol. 41, no. 12, 27 October 2020 (2020-10-27) - 27 October 2020 (2020-10-27), pages 1539 - 1546, XP037308507, ISSN: 1671-4083, DOI: 10.1038/S41401-020-00554-8
- DEEPAK VENKATARAMAN ET AL: "In vitro evaluation of anticancer properties of exopolysaccharides fromLactobacillus acidophilusin colon cancer cell lines", IN VITRO CELLULAR & DEVELOPMENTAL BIOLOGY. ANIMAL, SPRINGER US, NEW YORK, vol. 52, no. 2, 10 December 2015 (2015-12-10), pages 163 - 173, XP035943734, ISSN: 1071-2690, [retrieved on 20151210], DOI: 10.1007/S11626-015-9970-3

## Description

### Field of invention

The invention relates to particular species, strains, and compositions of lactic acid bacteria capable of increasing cellular levels of the hypoxia-inducible factor HIF-1 α for the treatment of hypoxia in hypoxia-inducing conditions such as chronic fatigue, oxygen deficiency, neurodegenerative diseases, pulmonary implications associated with respiratory failure, neonatal hypoxia-ischemia, myocardial ischemia, metabolic disorders, chronic cardiac and renal diseases, reproductive disorders such as pre-eclampsia and endometriosis, exacerbation of postural and kinetic tremors, and cerebral hypoxia.

### Background of the invention

Oxygen (O₂) is an essential nutrient that serves as a key substrate in cellular metabolism and energy production by aerobic organisms. In a variety of physiological and pathological states, organisms experience limited/insufficient O₂ disponibility, a condition referred to as hypoxia.

O₂ deprivation creates significant stress in living cells. This condition is related to the inappropriate accumulation of free radicals, which cause further stress on the protein component of cells and the genetic material they contain. To cope with the hypoxic stress condition, cells activate a series of adaptive responses to match O₂ supply with metabolic, bioenergetic, and redox demands. In particular, they temporarily arrest the cell cycle, reduce energy consumption, and secrete survival and pro-angiogenic factors. The intestinal mucosa receives between 10% and 35% of the total cardiac output, and the estimated surface area of the gastro-intestinal tract is about 250-300 m² under normal conditions (Lundquist et al., 2016). The gut is characterized by a peculiar oxygenation profile resulting from a combination of different factors, including massive fluctuations in blood perfusion as a result of food ingestion (Matheson et al., 2000). Changes in the amount of blood reaching the gut greatly affect the amount of O₂ available to the remaining body districts. The gut, therefore, plays a key role in determining the distribution of total O₂ available to the organism.

In the small intestine, increased oxygen availability sustains intense energy expenditure by highly proliferative stem cells and differentiated post-mitotic cells with high energy demand due to digestive, secretory and absorption processes (Rangel-Huerta et al., 2017; Van Der Schoor et al., 2002). The characteristics of oxygenation and oxygen consumption in the small intestine allow us to hypothesize that their modulation may have a major impact on the redistribution of globally available O₂ to the body. Under basal physiological conditions, intestinal mucosal epithelial cells are subject to relatively low O₂ levels, previously described as "physiological hypoxia" (Karhausen et al., 2005). To this condition, intestinal epithelial cells continuously adapt (Shepherd, 1982; Albenberg et al., 2014).

Hypoxia-inducible factors (HIFs) constitute key mediators of intestinal epithelial adaptation to its O₂-poor microenvironment (Ramakrishnan et al., 2016). These mediators are responsible for the reduction of oxygen consumption in mitochondria through inhibition of pyruvate to acetyl CoA conversion, suppression of mitochondrial biogenesis, and activation of mitochondria autophagy (Goda and Kanai, 2012). The reduction in cellular oxygen consumption associated with HIFs and the subsequent redistribution of oxygen in the peri-cellular microenvironment are supported by evidence produced using PHD inhibitors (Susser et al., 2020). HIFs are heterodimers composed of two subunits termed alpha and beta, respectively, the second of which is constitutively expressed by eukaryotic cells. The HIF-α subunit belongs to the helix-loop-helix Per-Arnt-Sim (bHLH-PAS) family of basic transcription factors (Schito et al., 2016). Vertebrates possess three subunits α: HIF-1α, HIF-2α and HIF-3α. The N-terminal region of these subunits contains a domain required for DNA binding and heterodimerization (Wu et al., 2015). The HIF-α subunits possess a highly conserved oxygen-dependent degradation (ODD) domain. The ODD domain contains two hydroxylated prolines, in both HIF-1α and HIF-2α (Chan et al., 2005). Hydroxylation of HIF-α leads to proteosomal degradation. The HIF-α subunits are hydroxylated by specific enzymes PHD1 (EGLN2), PHD2 (EGLN1) and PHD3 (EGLN3) belonging to the group of prolyl hydroxylase domain (PHD) enzymes that represent the main oxygen sensors in a cell. Under normoxic conditions, PHDs use O₂ to hydroxylate HIF-α subunits at the proline level present in the ODD. Hydroxylation allows the binding of the Von Hippel-Lindau oncosuppressor protein (VHL), which acts as an E3 ubiquitin ligase enabling the degradation of HIF-α (Ivan et al., 2001). Under conditions of low oxygen disponibility, PHD enzymes are unable to perform hydroxylation of HIF-α which are stabilized by heterodimerization with an HIF-β subunit (Wang et al., 1995). The generated heterodimer is able to bind genetic elements, termed HIF response elements (HREs) which are present within the promoters of target genes. Consequent to such binding, the target genes are expressed allow the cell to generate an adaptive response to the hypoxic condition (Toescu et al., 2004; Wiener et al., 1996). Although HIF-1α and HIF-2α are closely related and able to activate HRE-dependent expression, both subunits differ in their transactivation domains, implying that they have different gene targets. In particular, scientific evidence has shown that HIF-1α preferentially induces the glycolytic pathway and thus adaptation to energy production in O₂ deficiency, (Hu et al., 2003). Adaptation to hypoxia associated with HIF activity involves a number of changes in cellular metabolism. Chief among these is the reduction of oxygen consumption by shifting energy production from mitochondrial oxidative phosphorylation to anaerobic glycolysis. Within the intestinal environment, the regulation of HIFs is influenced by multiple factors associated with both cellular metabolism and microbial action (Singhal et al., 2020).

Hypoxic conditions are frequently observed in the presence of a wide variety of pathological conditions, acute and chronic. Hypoxia-associated pathologies include neonatal hypoxia-ischemia, myocardial ischemia, metabolic disorders, chronic cardiac and renal pathologies, reproductive disorders such as preeclampsia and endometriosis, exacerbation of postural and kinetic tremors, cerebral hypoxia, and neurodegenerative diseases (Chen et al., 2020; Legros et al., 2010; Nalivaeva et al., 2019; Merelli et al., 2020). Hypoxia assumes relevant importance in pathological conditions resulting from loss of lung surface respiratory function. In this context, the condition of acute respiratory distress caused by infections of the new pandemic coronavirus Sars-CoV-2 (Gibson et al., 2020; Ramirez et al., 2020) should be highlighted. In addition, hypoxic conditions are closely related to the onset of altered physiological conditions and pathological manifestations associated with prolonged stay in conditions of low O₂ availability. In this context, it is incumbent to highlight the pulmonary, nervous, and muscular impairments associated with activities performed at high altitude. Spagnolello et al. ("Targeting Microbiome: An Alternative Strategy for Fighting SARS-CoV-2 Infection", CHEMOTHERAPY, vol. 66, no. 1-2 1 January 2021 (2021-01-01), pages 24-32, ISSN: 0009-3157, DOI: 10.1159/000515344) discloses that respiratory and gastrointestinal symptoms are the predominant clinical manifestations of the coronavirus disease 2019 (COVID-19), which infects intestinal epithelial cells and may impact on host's microbiota and gut inflammation. Since an imbalanced intestinal microbiome can affect pulmonary function, modulating the host immune response ("gut-lung axis"), addressing intestinal dysbiosis with a probiotic supplement may be an option for preventing and treating COVID-19 pneumonia. EP2455092 discloses compositions comprising non-replicating probiotic micro-organisms for use in the prevention or treatment of upper respiratory tract infections and/or their symptoms. In contrast to reports in the scientifical literature for probiotic microorganisms (Esfandiary et al., 2016; Deepak et al., 2015; Chen et al., 2020; Han et al, 2020), the Applicant surprisingly found that oral administration of *Lactobacillus acidophilus,* or *Lactobacillus acidophilus* and *Streptococcus thermophilus* and/or *Bifidobacterium animalis* subsp. *lactis,* preferably with the addition of *Levilactobacillus brevis* (formerly known as *Lactobacillus brevis), Lactiplantibacillus plantarum* subsp. *plantarum* (formerly known as *Lactobacillus plantarum*), *Lactobacillus helveticus, Lacticaseibacillus paracasei* subsp. *paracasei* (formerly known as *Lactobacillus paracasei* subsp. *paracasei*) is capable of increasing the expression/stabilization of HIF-1α and that particular species, strains, and compositions of lactic acid bacteria are therefore capable of maintaining or enhancing normoxia under hypoxia-inducing conditions such as, e.g., chronic fatigue, oxygen deprivation, or for the treatment of hypoxia in conditions involving hypoxia such as neurodegenerative diseases, pulmonary implications associated with respiratory failure, neonatal hypoxia-ischemia, myocardial ischemia, metabolic disorders, chronic cardiac and renal diseases, reproductive disorders such as preeclampsia and endometriosis, exacerbation of postural and kinetic tremors, and cerebral hypoxia.

### Compendium of the Invention

An object of the invention is the strain of *Lactobacillus acidophilus* deposited by the Applicant under the Budapest Treaty on September 1, 2020 at the Collection Nationale de Cultures de Microorganismes, Institut Pasteur, 25 rue du Docteur Roux, 75724 Paris Cedex 15, and having the accession number CNCM I-5567 capable of positively modulating cellular levels of the hypoxia-inducible factor HIF-1α associated with the reduction of cellular oxygen consumption for use in the treatment of hypoxia-inducing conditions such as, for example, chronic fatigue, oxygen deficiency, or for use in the treatment of hypoxia in conditions involving hypoxia such as neurodegenerative diseases, pulmonary implications associated with respiratory failure, neonatal hypoxia-ischemia, myocardial ischemia, metabolic disorders, chronic cardiac and renal diseases, reproductive disorders such as preeclampsia and endometriosis, exacerbation of postural and kinetic tremors, and cerebral hypoxia.

A further object of the invention is a composition comprising the aforementioned *Lactobacillus acidophilus* and optionally one or more pharmaceutically acceptable excipients for the above-mentioned use.

According to one aspect of the invention, the composition further comprises the strain of *Streptococcus thermophilus* deposited by the Applicant under the Budapest Treaty on September 1, 2020 at the Collection Nationale de Cultures de Microorganismes, Institut Pasteur, 25 rue du Docteur Roux, 75724 Paris Cedex 15, and having the CNCM accession number I-5570, and the strain of *Bifidobacterium animalis* subsp. *lactis* deposited by the Applicant pursuant to the Budapest Treaty on September 1, 2020 at the Collection Nationale de Cultures de Microorganismes, Institut Pasteur, 25 rue du Docteur Roux, 75724 Paris Cedex 15, and having access number CNCM I-5571 and/or the strain of *Bifidobacterium animalis* subsp. *lactis* deposited by the Applicant under the Budapest Treaty on September 1, 2020, at the Collection Nationale de Cultures de Microorganismes, Institut Pasteur, 25 rue du Docteur Roux, 75724 Paris Cedex 15, and having accession number CNCM I-5572 for the above-mentioned use.

According to a further aspect of the invention, the composition includes from 30% to 50% by weight of *Lactobacillus acidophilus,* from 25% to 35% by weight of *Streptococcus thermophilus,* and from 25% to 35% by weight of *Bifidobacterium animalis* subsp. *lactis,* depending on the weight of the composition for the above-mentioned use.

According to a further aspect, the composition additionally includes the strain of *Levilactobacillus brevis* deposited by the Applicant under the Budapest Treaty on September 1, 2020 at the Collection Nationale de Cultures de Microorganismes, Institut Pasteur, 25 rue du Docteur Roux, 75724 Paris Cedex 15, and having the accession number CNCM I-5566, the strain of *Lactiplantibacillus plantarum* subsp. *plantarum* deposited by the Applicant under the Budapest Treaty on September 1, 2020 at the Collection Nationale de Cultures de Microorganismes, Institut Pasteur, 25 rue du Docteur Roux, 75724 Paris Cedex 15, and having accession number CNCM I-5569, the strain of *Lacticaseibacillus paracasei* subsp. *paracasei* deposited by the Applicant under the Budapest Treaty on September 1, 2020, at the Collection Nationale de Cultures de Microorganismes, Institut Pasteur, 25 rue du Docteur Roux, 75724 Paris Cedex 15, and having accession number CNCM I-5568, and the strain of *Lactobacillus helveticus* deposited by the Applicant under the Budapest Treaty on September 1, 2020, at the Collection Nationale de Cultures de Microorganismes, Institut Pasteur, 25 rue du Docteur Roux, 75724 Paris Cedex 15, and having accession number CNCM I-5573 for the above-mentioned use.

According to a further aspect, the composition includes from 30% to 50% by weight *Lactobacillus acidophilus,* from 1% to 10% by weight *Streptococcus thermophilus,* from 1% to 20% by weight *Bifidobacterium animalis* subsp. *lactis,* from 1% to 10% by weight *Levilactobacillus brevis* (formerly known as *Lactobacillus brevis),* from 1% to 10% by weight *Lactiplantibacillus plantarum* subsp. *plantarum* (formerly known as *Lactobacillus plantarum*), from 1% to 10% by weight of *Lacticaseibacillus paracasei* subsp. *paracasei* (formerly known as *Lactobacillus paracasei* subsp. *paracasei*), and from 1% to 10% by weight of *Lactobacillus helveticus,* depending on the weight of the composition for the above-mentioned use.

According to a further aspect, the composition according to the invention is suitable for oral administration, such as in the form of powders, capsules or granules for the above-mentioned use. The composition preferably has a high concentration of bacteria, to the extent of at least 10 billion in the adult and at least 100 million in the infant for the above-mentioned use.

According to a further aspect, the composition according to the invention is suitable for oral administration, such as in the form of powders, capsules, granules or spray, to animals reared and/or maintained in oxygen-deficient conditions, for example, on farms located at relevant heights above sea level.

The used probiotic bacteria may be viable, nonviable, sonicated, tindalized or lyophilized.

Finally, a method is disclosed (which does not form part of the invention) for identifying *Lactobacillus acidophilus* capable of positively modulating cellular levels of the hypoxia-inducible factor HIF-1α associated with the reduction of cellular oxygen consumption that includes the steps:
evaluation, by western blot technique, of cellular levels of HIF-1α in *in vitro* cellular models suitable for proper physiological representation of the target tissue in the presence and absence of treatment of at 24 hours with bacterial lysate specific to the *Lactobacillus acidophilus* strain to be evaluated; and
evaluation, in the same cellular model, of the extracellular acidification rate (ECAR), oxygen consumption rate (OCR), and the relative glycolysis rate (ECAR/OCR ratio) in the presence and absence of treatment of at 24 hours with bacterial lysate specific to the *Lactobacillus acidophilus* strain to be evaluated, using the Seahorse XFe96 analyzer (Agilent) according to the manufacturer's instructions or equivalent technique.

### Brief Description of Figures

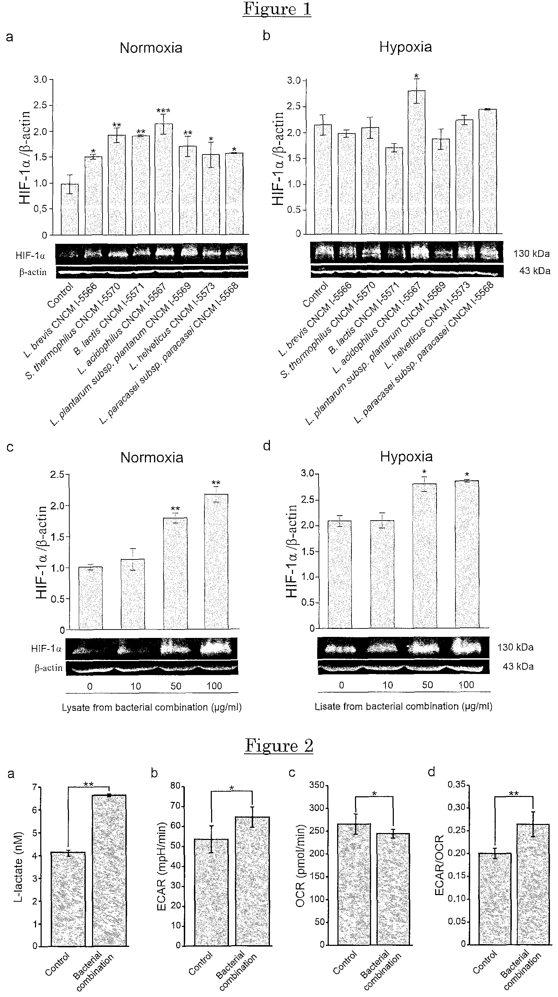

Figure 1 shows the effect of treatment with probiotic strains on HIF-1α levels under normoxic and hypoxic conditions. Western blotting for HIF-1α on differentiated Caco-2 cells for 6 days incubated for 24 under normoxic and hypoxic conditions: a), b) in the presence or absence of the soluble fraction of bacterial lysates of strains *Levilactobacillus brevis* CNCM I-5566, *Lactobacillus acidophilus* CNCM I-5567, *Lactiplantibacillus plantarum* subsp. *plantarum* CNCM I-5569, *Lactobacillus helveticus* CNCM I-5573, *Lacticaseibacillus paracasei* subsp. *paracasei* CNCM I-5568, *Bifidobacterium animalis* subsp. *lactis* CNCM I-5571, and *Streptococcus thermophilus* CNCM I-5570 (100 µg/ml); (c), (d) in the presence or absence of a specific combination of probiotic strains, comprising the bacterial strain *Bifidobacterium animalis* subsp. *lactis* CNCM I-5572 in addition to those listed above. Following densitometric analysis, the values obtained were normalized with respect to β-actin. Data shown are expressed as mean ± SD of a duplicate experiment. Data were compared by one-way analysis of variance (ANOVA) followed by Dunnet's test. * p < 0.05, ** p < 0.01, *** p < 0.001. A representative immunoblot showing the quantification of HIF-1α and β-actin is also shown in the figure.
Figure 2 shows the effect of treating Caco-2 cells with bacterial lysates from the probiotic strain mixture on (a) L-lactate levels, (b) the extracellular acidification rate (ECAR) (c) the oxygen consumption rate (OCR) and (d) the ECAR/OCR ratio. Caco-2 cells were treated with or without bacterial lysate (100 µg/ml) for 24 hours. Lactate levels were analyzed by colorimetric assay. ECAR and OCR values were obtained by Seahorse XF extracellular flow analyzer. Values are expressed as mean ± SEM of three independent experiments in triplicate. For comparison between two averages, the Student's t test for unpaired data was used. (*p<0.05; **p<0.01).

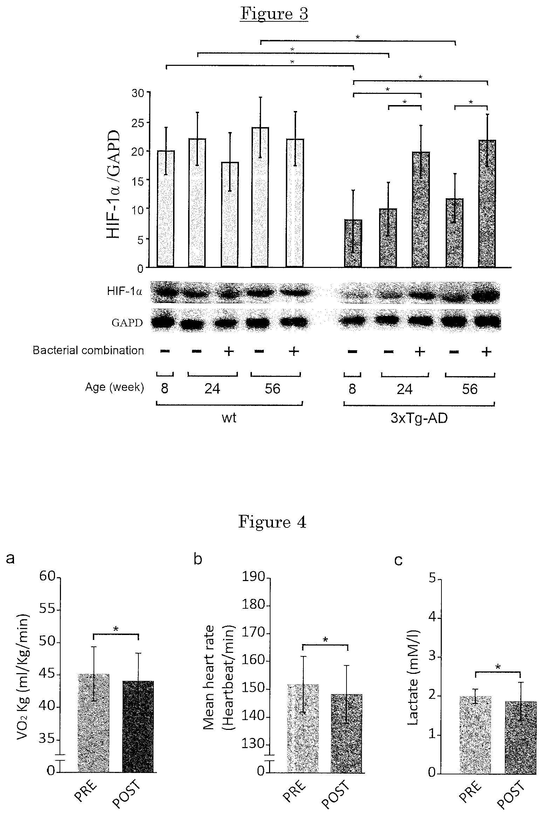

Figure 3 shows the effect of treatment with the above mixture of probiotic strains on HIF-1α levels in brain homogenates of wild-type (wt) mice and 3xTg-AD mice used as a mouse model of Alzheimer's disease. Analyses were conducted on brain extracts from guinea pigs sacrificed at 8 weeks of age and after 16 and 48 weeks after treatment initiation. Following densitometric analysis, the obtained values were normalized against GlycerAldehyde-3-Phosphate Dehydrogenase (GAPD). Data shown are expressed as mean ± SD of three experiments in duplicate. Data were compared by one-way analysis of variance (ANOVA) followed by Bonferroni's test. * p <0,05. A representative immunoblot showing quantification of HIF-1α and GAPD is also shown in the figure.
Figure 4. shows the effect of taking a specific probiotic formulation on (a) oxygen consumption, (b) average heart rate in the last 5 minutes of exercise, and (c) blood lactate levels, in subjects practicing endurance sports. Data shown are expressed as mean ± SD of a triplicate experiment. Statistical significance between the group of trials conducted in the absence of probiotic intake and those conducted after taking a specific oral bacteriotherapy was determined by Student's t-test for paired data. *: p≤0,05.

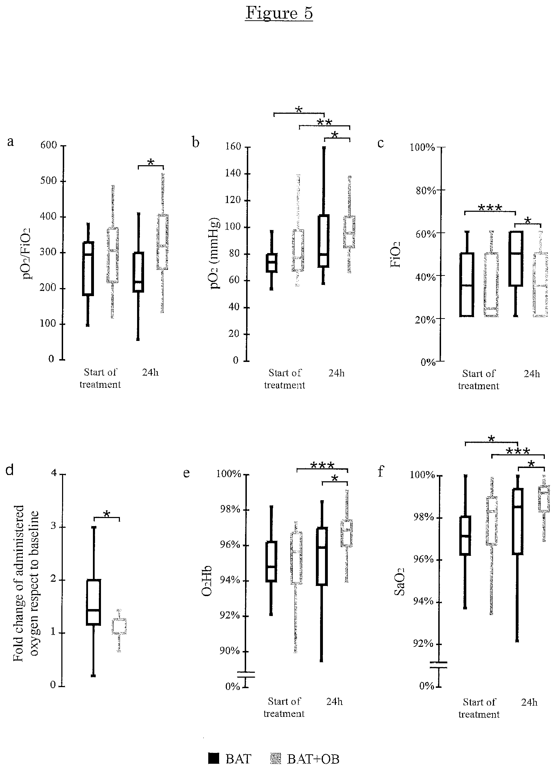

Figures 5 show box whisker graphs illustrating the distribution of the value of the considered blood parameters, obtained from routine laboratory analysis. Where present, statistical significance was reported between the MTD and MTD+BO groups at each time point as well as for each group over time. *: p≤0.05; **: p≤0.001; ***: p≤0.0001.

### Detailed Description of the Invention

### In vitro study

HIF-1α represents a key mediator in the regulation of oxygen homeostasis and preferential induction of the glycolytic pathway and thus adaptation to energy production in O₂ deficiency resulting in reduced oxygen consumption by shifting energy metabolism to that pathway (Hu et al., 2003).

The inventor conducted *in vitro* assays on intestinal-derived cellular models in order to evaluate the ability of specific bacterial strains alone or in combinations in modulating HIF-1α accumulation associated with the reduction of cellular oxygen consumption. Obtained results show that, under normoxic conditions, the exposure of Caco-2 cells to bacterial lysates of *L. brevis* CNCM I-5566, *L. acidophilus* CNCM I-5567, *L. plantarum* CNCM I-5569, *L. helveticus* CNCM I-5573, *L. paracasei* CNCM I-5568, *B. lactis* CNCM I-5571 and *S. thermophilus* CNCM I-5570 is associated with a significant increase in intracellular HIF-1α levels compared with the untreated control (Figure 1a). *S. thermophilus* CNCM I-5570, *B. lactis* CNCM I-5571 and *L. acidophilus* CNCM I-5567 were the most effective (~2.2-fold increase for *L. acidophilus* CNCM I-5567 and ~2-fold increase for *S. thermophilus* CNCM I-5570 and *B. lactis* CNCM I-5571). In hypoxic conditions, the strains did not induce significant changes compared to untreated cells, except for *L. acidophilus* CNCM I-5567 whose addition resulted in a significant increase in HIF-1α levels compared to control (Figure 1b). Exposure of Caco-2 cells to combined bacterial extracts at the concentration of 50 and 100 µg/ml was associated with a significant increase in cellular accumulation of HIF-1α under both normoxic and hypoxic conditions (Figure 2a and b). It is noteworthy that under normoxic conditions, the levels of HIF-1α accumulation recorded for the combined bacterial lysate at the concentration of 100 µg/ml were comparable to those determined at the same concentration for the lysate related to *L. acidophilus* CNCM strain I-5567 alone. Under hypoxic conditions, already at the concentration of 50 µg/ml, the bacterial lysate related to the combination of probiotic strains induced a cellular accumulation of HIF-1α similar to that recorded for *L*. *acidophilus* strain CNCM I-5567 alone at higher concentrations. Considering that the *L. acidophilus* strain CNCM I-5567 represents a quantitatively minority fraction of the bacteria present within the probiotic combination, the observed results suggest that the combined use of the specific probiotic strains tested is characterized by a synergistic effect with respect to the induction of cellular accumulation of HIF-1α.

The influence of lysed bacterial strains on cellular energy metabolism and cellular oxygen consumption was investigated. To this end, levels of L-lactate, a key metabolite of the glycolysis pathway; the extracellular acidification rate of the medium (ECAR), a parameter reflecting glycolysis; and the oxygen consumption rate (OCR) used to determine oxidative phosphorylation were assessed within the culture media. As a result of exposing the Caco-2 cell line to total bacterial lysate for 24 hours, there were significantly lower OCR values attesting to a reduction in cellular oxygen consumption compared to the untreated control (Figure 2c). In contrast, exposure of the cell line to bacterial lysate was associated with a significant increase in Lactate levels, ECAR values and ECAR/OCR ratio attesting to increased glycolysis (Figure 2a, 2b and 2d).

### Conclusions

The amount of oxygenated blood recalled at the intestinal level conditions the availability of O₂ in extraintestinal body districts including essential organs such as brain, heart, kidney, and liver.

In the intestine, oxygen homeostasis is largely dependent on HIFs. Probiotic microorganisms have the potential to modulate HIFs and influence the processes regulated by them. Strains belonging to the bacterial species *L. paracasei, L. acidophilus, L. crispatus, L. rhamnosus,* and *B. longum* are able to inhibit, *in vitro,* the expression of HIF-1α in various cellular models (Han et al., 2020; Esfandiary et al., 2016; Deepak et al., 2015; Chen et al., 2020). Contrary to reports in the literature, the inventor's results showed, surprisingly, that the tested probiotic microorganisms are able to positively modulate the accumulation of HIF-1α. This contrasting effect, finds an explanation in the fact that the regulation of HIF-1α reflects the involvement of different molecular mechanisms. In addition, the beneficial effect produced by probiotics depends on the specific physiological state of host cells (McFarland et al., 2018). The increased accumulation of HIF-1α induced by the tested bacterial species is associated with a significant reduction of oxygen consumption in intestinal cells and the induction of anaerobic metabolism, which allows their survival under conditions of scarcity of this gas. The oxygen sparing induced by the tested bacteria could modulate the consumption of such gas in the intestine. The amount of O₂ not consumed in that body district could become available to other essential organs and tissues.

### Materials and methods

### Cell cultures and treatments

The human colon adenocarcinoma cell line (Caco-2) was cultured in DMEM (Dulbecco's Modified Eagle's Medium) medium containing 10% (v/v) fetal bovine serum, 1% nonessential amino acids, 1 mM sodium pyruvate, 2 mM glutamine, 100 U/ml penicillin and 100 µg/ml streptomycin and incubated in a humidified atmosphere with 5% CO₂ at 37°C. After reaching 80% confluence, the cells were detached and plated in a multiwell plate of 6 at the concentration of 6x10⁴ cells/cm². Cell growth was monitored by light microscopy. For assessment of cellular HIF-1α levels of extracellular acidification rate and oxygen consumption rate, cells differentiated at 14 days post-confluence were pretreated with or without the indicated concentration of probiotic for 30 min and then incubated in normoxia under standard culture conditions, (~21% O₂) or in hypoxia using a "hypoxia incubation chamber" (1% O₂) for 24 hr.

### Preparation of the soluble fraction of bacterial lysates

The soluble fraction of bacterial lysates was prepared as follows: each sample was washed three times (8,600× g for 20 min at 4°C) and resuspended in phosphate buffered saline (PBS). The bacterial suspension was sonicated for 30 minutes, alternating 10 seconds of sonication and 10 seconds of pause, and centrifuged at 17.949× g for 20 minutes at 4°C. The supernatant was filtered through a 0.22 µm filter so as to remove any remaining intact bacteria, and the protein concentration was determined. For tests on individual bacterial strains, the concentration of the soluble fraction of the bacterial lysate was determined to be 100 µg/ml. The assays concerning probiotic strain combinations, were performed with increasing concentrations of total bacterial lysate soluble fraction of 10, 50 and 100 µg/ml, respectively. The assays performed to evaluate the action of the combination of strains was carried out on a probiotic formulation in which the percentage of bacterial cells of each individual strain to total bacterial cells in the compound was as follows: 35.46% *L. brevis* CNCM I-5566, 1.42% *L. acidophilus* CNCM I-5567, 5.32% *L. plantarum* CNCM I-5569, 0.71% *L. helveticus* CNCM I-5573, 2.13% *L. paracasei* CNCM I-5568, 17.73% *B. lactis* CNCM I-5571, 1.77% *B. lactis* CNCM I-5572 and 35.46% *S. thermophilus* CNCM I-5570 . The samples thus prepared were frozen at -80°C until use. Untreated cells were considered as controls.

### Western blot

HIF-1α expression was assessed by Western blotting. Cells were lysed on ice using RIPA buffer containing protease inhibitors for 30 min on ice. After cell lysis, samples were centrifuged at 17,949× g for 20 min at a temperature of 4°C. The supernatant was recovered and the total protein assay was performed. Sample buffer and mercaptoethanol was added to a volume of supernatant equivalent to 25 g of protein, and samples were boiled for 5 min and separated by sodium dodecyl sulfate (SDS)-polyacrylamide 10% gel electrophoresis (SDS-PAGE). Transfer of samples onto nitrocellulose membrane (0.45 µm) was carried out at constant 70 volts for 1 h at 4°C, and the nitrocellulose filter was incubated at room temperature for 1 h with aspecific siteblocking solution and then incubated overnight at 4°C with monoclonal anti-HIF-1α or anti-β-actin antibody. After incubation with the secondary antibody, conjugated with horseradish peroxidase (HRP), the immunoreactive bands were visualized by chemiluminescence. Densitometric analysis of the bands corresponding to HIF-1α was then performed, and the values obtained were normalized to those of β-actin.

### L-lactate production assays

L-lactate levels in cell culture supernatants were assayed using the L-lactate assay kit (Abcam, Cambridge, UK) according to the manufacturer's instructions. Supernatants were deproteinized with a 10-kDa NMWCO centrifugal filtration unit (Amicon, Millipore), and the filtrate was added to reaction wells. Absorbance was measured by spectrophotometric reading at 570 nm.

### Metabolic studies

Cells treated as described above were evaluated for extracellular acidification rate (ECAR) and oxygen consumption rate (OCR) to calculate the rate of glycolysis (ECAR/OCR) using the Seahorse XFe96 analyzer (Agilent) following the manufacturer's instructions. Briefly, on the day of the test, the medium was changed to Seahorse XF DMEM Medium pH7.4 supplemented with glucose (10 mmol/L), pyruvate (1 mmol/L) and glutamine (2 mmol/L) (Agilent), and the cells were allowed to equilibrate in a non-CO₂ incubator for 1 h; OCR and ECAR were then measured. XFp Mito Stress Test Kit was used to test mitochondrial function. Injection of oligomycin (1 pM), carbonyl cyanide-4 (trifluoromethoxy) phenylhydrazone (FCCP, 1 pM), and the mixture of rotenone and antimycin A (1 pM) allowed the determination of key bioenergetic parameters: basal respiration, ATP production-related respiration (ATP production), maximal respiration, reserve respiratory capacity, nonmitochondrial respiration, proton leakage, and coupling efficiency.

### Statistical Analysis.

ANOVA test followed by Dunnett's or Tuckey post hoc tests was used to check for statistically significant differences between the different conditions tested while, in the case of two groups, comparisons between mean values were made by unpaired Student's t test. A p value ≤0.05 was considered statistically significant. Analyses were performed using R 4.0.3 statistical software.

### In vivo study

Reduced oxygen supply to the brain plays a key role in neurodegeneration during the aging process (Ogunshola and Antoniou, 2009). Pathological processes such as oxidative stress, impaired oxygen or glucose supply, and disruption of iron homeostasis are common in neurodegenerative diseases (Correia and Moreira, 2010; Gironi et al., 2011; Benarroch, 2009). Reduced brain levels of HIF-1α, associated with decreased expression of GLUT1 and GLUT2 receptors responsible for glucose uptake, have been previously demonstrated in models of Alzheimer's disease (AD). The inventor conducted *in vivo* assays in order to evaluate the ability of a specific bacterial combination in modulating HIF-1α accumulation in brain tissue of 3xTg-AD mice. This reliable model of human AD shows both plaque and tangle pathology, with intracellular Aβ immunoreactivity detectable at three months of age and hyperphosphorylation of tau protein occurring between 12 and 15 months of age (Oddo et al., 2003). The characteristics of the experimental design, as well as, the preparation of brain extracts were in line with what was previously reported in 2018 by Bonfili et al. (Bonfili et al., 2018). Forty-eight 3xTg-AD guinea pigs, eight weeks old, were divided into 2 groups. The first group (n=24) was treated with a specific probiotic formulation containing *Streptococcus thermophilus* CNCM I-5570, *Bifidobacterium animalis* subsp. *lactis* CNCM I-5571, *Bifidobacterium animalis* subsp. *lactis* CNCM I-5572, *Lactobacillus acidophilus* CNCM I-5567, *Lactobacillus helveticus* CNCM I-5573, *Lacticaseibacillus paracasei* subsp. *paracasei* CNCM I-5568, *Lactiplantibacillus plantarum* subsp. *plantarum* CNCM I-5569 and *Lactobacillus brevis* CNCM I-5566 while the other (n=24), untreated, was used as a control. Simultaneously, 48 wild-type (wt) mice of the same age were also divided into 2 groups of equal numbers, only one of which was treated with the same probiotic formulation. The dosage (200 billion bacteria/kg/day) was determined using body surface area normalization as previously reported in the literature (Crawford et al., 1950). Mice were sacrificed for biochemical analysis at 24 and 56 weeks of age (16 and 48 weeks from the start of treatment), and brains were stored at -80°C until brain homogenates were produced. ANOVA followed by Bonferroni's test was used to test for statistically significant differences in HIF-1α expression levels. A p value≤0.05 was considered statistically significant. The level of HIF-1α subunit in brain homogenates was analyzed by Western blotting assay as previously described (Bonfili et al., 2018). The results obtained showed that untreated 3xTg-AD mice had significantly lower brain levels of HIF-1α than wt mice of the same age. Surprisingly, administration of a probiotic formulation in 3xTg-AD mice was associated with a significant increase in brain levels of HIF-1α. Unexpectedly, treatment with the probiotic restored HIF-1α expression to the levels recorded for wt mice of equal age (Figure 3). The increased brain levels of HIF-1α observed suggest that administration of the specific probiotic formulation tested could improve oxygen homeostasis and glucose metabolism in the brain constituting a viable therapeutic approach for the treatment of neurodegenerative diseases.

### Studies performed on humans

### First study on humans

In this study, the effect of taking a specific probiotic formulation on respiratory, cardiac, and metabolic parameters in subjects practicing endurance sports was evaluated. For this purpose, 4 male Triathlon practicing subjects (Age: mean±DS, 37±5 years; Weight: mean±DS 70±3 kg) were recruited. Two sets of trials were carried out, the first of which denoted "PRE" involved carrying out the protocol in the absence of intake of specific probiotic formulation. In the second set of trials, called "POST," the same subjects repeated the test after intake of a bacterial composition consisting of *Streptococcus thermophilus* CNCM I-5570, *Bifidobacterium animalis* subsp. *lactis* CNCM I-5571, *Bifidobacterium animalis* subsp. *lactis* CNCM I-5572, *Lactobacillus acidophilus* CNCM I-5567, *Lactobacillus helveticus* CNCM I-5573, *Lacticaseibacillus paracasei* subsp. *paracasei* CNCM I-5568, *Lactiplantibacillus plantarum* subsp. *plantarum* CNCM I-5569 and *Lactobacillus brevis* CNCM I-5566. The amount of probiotics taken consisted of the ingestion of about 400 billion bacterial cells in a single dose. Subjects were asked to consume their last meal with dinner on the day before the tests and to appear fasting at their assigned time, allowing water intake only; in the "POST" test sets, they were asked to take the probiotic formulation no earlier than 5 hours after the last meal and at least 5 hours before the time of the test. The tests were conducted midweek to minimize the impact of physical activity during the weekend. Each subject repeated the two sets of tests on the same day and at the same time. The tests were performed on a Panatta Treadmill model T-190 (Panatta, Italy) with a fixed belt incline of 1%. After subjects were made to wear the metabolimeter mask, they were allowed to perform a 5' warm-up at free intensity, but lower than the test intensity. Successively, without interruption the intentionality was increased to a threshold value for another 10'. This value was chosen using individual subjects' knowledge of their own anaerobic threshold intensity and represented an appropriate exercise intensity for running a total distance of 20 km. Measurements of the average heart rate over the last 5 minutes of exercise and the amount of oxygen that the body is able to extract and then use in the unit of time for muscle contraction (VO₂) were acquired using Fitmate PRO equipment (COSMED, Italy) interfaced with a heart rate monitor band (POLAR, Italy). Blood lactate concentration was determined by capillary sampling from the earlobe performed at the end of the threshold intensity step. With respect to the parameters considered, the presence of significant differences between the PRE and POST test groups was assessed by Student's t test for paired data. A p value ≤0.05 was considered statistically significant. Lactate represents the end product of the glycolytic cycle when it is carried out in oxygen deficiency so its concentration reflects the level of anaerobic metabolism. Heart rate and VO₂ constitute additional parameters that can give indications of the level of aerobic metabolism as a reduction in these parameters indicates an improvement in aerobic metabolism and thus greater oxygen availability. In general, the reduction in heart rate, VO₂ and blood lactate concentration suggest an improvement in the efficiency of aerobic metabolism associated with the intake of the probiotic formulation (Figure 4).

These results are consistent with the hypothesis that the positive modulation of HIF-1α, induced by the action of specific probiotic strains in the intestine, would allow a reduction in O₂ consumption in that district. The oxygen sparing induced by probiotic intake would cause this gas to be redistributed by becoming more available in the blood circulation and consequently to other body districts.

### Second study on humans

Hypoxia is a common condition in many disease states that takes on particular relevance in conditions associated with acute lung injury (Lee et al., 2019). The purpose of the present work was to investigate the baseline action performed by bacterial strains in alleviating respiratory conditions in subjects with pulmonary implications associated with Sars-CoV-2 infections. In addition, the earliness with which this beneficial effect appear evident was evaluated. To this aim, responses of two groups of patients was examined, one group was treated with the best available therapy (BAT), while the other was additionally supplemented with oral bacteriotherapy (BAT+OB). The effect of probiotic intake was evaluated by comparing the blood oxygenation parameters partial pressure of oxygen (pO₂), fraction of inspired oxygen (FiO₂), oxygenated hemoglobin (O₂Hb), pO₂/FiO₂ ratio, and oxygen-saturated hemoglobin (SaO₂) of the two groups at the beginning of treatment and in the following twenty-four hours. The amount of oxygen delivered (l/min) was additionally measured. The main characteristics of both groups of patients are summarized in Table 1.

**Table 1**

| **Parameter** | **BAT (No./%= 29/42%)** | | **BAT+OB (No/%.=40/58%)** | | **p Value** |
|---|---|---|---|---|---|
| | Median (IQR) | No. (%) | Median (IQR) | No. (%) | |
| Age (year) | 70 (60-77) | | 61 (51-74.3) | | 0.09 |
| Sex (Males) | | 25 (86.2) | | 22 (55) | 0.01 |
| BMI - kg/m³ | 20 (20-22) | | 20 (18.8-22) | | 0.47 |
| ALT - IU/l | 25 (18-40) | | 30 (23.5-45) | | 0.17 |
| AST - IU/l | 21 (19-38) | | 26 (18-36.3) | | 0.95 |
| Charlson index | 3 (1-4) | | 1 (1-5) | | 0.24 |
| Drug therapy | | | | | |
| Antivirals (Remdesivir) | | 10 (34.4) | | 8 (20) | 0.28 |
| Antibiotics | | 25 (86.2) | | 39 (97.5) | 0.19 |

| | | | | | |
|---|---|---|---|---|---|
| IQR: Interquartile interval | | | | | |

With the exception of sex, the two groups determined by the administration of the probiotic formulation were homogeneous for all clinical considered variables including drug therapies for treatment of SARS-CoV-2 infections, blood oxygenation parameters and amount of oxygen administered (median; IQR BAT 4; 1-6 l/min; BAT+OB 1.5; 1-6 l/min, p = 0.31). Twenty-four hours after the first probiotic dose, the BAT+OB group showed significantly higher values of the pO₂/FiO₂ ratio and pO₂ than the BAT group while an opposite situation was observed for FiO₂ values (Figures 5a-c). Analysis of O₂Hb and SaO₂ levels produced results in line with those previously described for the pO₂ and pO₂/FiO₂ ratio (Figures 5e and 5f). Overall results showed that after 24 hours from the start of treatments, the group administered with the probiotic formulation had better blood oxygenation levels than the group taking only the standard therapy, although the BAT group experienced a significantly higher increase in the amount of oxygen delivered over time (Figure 5d).

The improvement in blood oxygenation parameters observed in the BAT+OB group are consistent with the hypothesis that, at the gut level, the positive modulation of HIF-1α induced by the action of specific probiotic strains would allow a reduction in O₂ consumption which would be redistributed by becoming more available at the level of the bloodstream.

### Patients and methods

### Study design, population of subjects, data collection and treatment

This study was performed on SARS-CoV-2 infected adult patients (>18 years), supported with oxygen therapy administered via Venturi mask under spontaneous breathing regimen. The diagnosis of SARS-CoV-2 infection was defined by a positive oropharyngeal and nasopharyngeal swab performed in duplicate for SARS-CoV-2 E and S gene by reverse transcriptase polymerase chain reaction (RT-PCR). Patients included in the study were housed in two different wards devoted to the management of COVID-19: in the first ward, only BAT was administered as suggested by the Società Italiana di Malattie Infettive e Tropicali (SIMIT) (Italian Society of Infectious and Tropical Diseases) and Italian Medicine Agency (AIFA) interim guidelines, comprising Dexamethasone (6 mg daily for 10 days) plus low molecular weight heparins (prophylactic dosage) +/- azithromycin (500 mg daily); Remdesevir, as per AIFA guidelines. In the second ward, BAT was combined with the administration of oral bacteriotherapy consisting of a total of 2,400 billion bacteria per day comprising *S. thermophilus* CNCM I-5570, *B. lactis* CNCM I-5571, *B. lactis* CNCM I-5572, *L. acidophilus* CNCM I-5567, *L. helveticus* CNCM I-5573, *L. paracasei* CNCM I-5568, *L. plantarum* CNCM I-5569, and *L. brevis* CNCM I-5566 strains. Considered variables included 1) medical history data, 2) past medical history (comorbidities), 3) current medical history, treatment, and laboratory data. Arterial blood gas analysis (ABG test) was performed using blood taken from the radial artery 24 hours after the start of treatments.

### Statistical analysis

Categorical variables including sex, antiviral drug therapy, and antibiotic administration were compared using the χ2 test with Yates continuity correction to account for the limited sample size and shown as absolute frequencies and percentages. The two-sided Mann-Whitney U-test was used for all continuous variables including respiratory variables (pO₂, FiO₂, pO₂/FiO₂ Change in oxygens supplied compared to treatment onset, O₂Hb, SaO₂), biochemical variables (blood glucose, lactates, hematocrit) and demographic and clinical variables (age, BMI, ALT, AST, Charlson Index) in order to determine statistically significant differences between groups at each considered time point while, for each group, the Wilcoxon test was used to assess significant differences between consecutive time points. In all cases a p-value≤0.05 was considered statistically significant.

### BIBLIOGRAFY

Albenberg L, Esipova TV, Judge CP, Bittinger K, Chen J, Laughlin A, Grunberg S, Baldassano RN, Lewis JD, Li H, Thom SR, Bushman FD, Vinogradov SA, Wu GD. Correlation between intraluminal oxygen gradient and radial partitioning of intestinal microbiota. Gastroenterology. 2014 Nov;147(5):1055-63.e8. doi: 10.1053/j.gastro.2014.07.020. Epub 2014 Jul 18. PMID: 25046162; PMCID: PMC4252572.

Benarroch EE. Brain iron homeostasis and neuro-degenerative disease. Neurology. 2009 Apr 21;72(16):1436-40. doi: 10.1212/WNL.0b013e3181a26b30. PMID: 19380704.

Bonfili L, Cecarini V, Cuccioloni M, Angeletti M, Berardi S, Scarpona S, Rossi G, Eleuteri AM. SLAB51 Probiotic Formulation Activates SIRT1 Pathway Promoting Antioxidant and Neuro-protective Effects in an AD Mouse Model. Mol Neurobiol. 2018 Oct;55(10):7987-8000. doi: 10.1007/s12035-018-0973-4. Epub 2018 Feb 28. PMID: 29492848; PMCID: PMC6132798.

Chan DA, Sutphin PD, Yen SE, Giaccia AJ. Coordinate regulation of the oxygen-dependent degradation domains of hypoxia-inducible factor 1 alpha. Mol Cell Biol. 2005 Aug; 25(15):6415-26. doi: 10.1128/MCB.25.15.6415-6426.2005. PMID: 16024780; PMCID: PMC1190339.

Chen C, Wang Y, Tang Y, Wang L, Jiang F, Luo Y, Gao X, Li P, Zou J. Bifidobacterium-mediated high-intensity focused ultra-sound for solid tumor therapy: comparison of two nanoparticle delivery methods. Int J Hyperthermia. 2020;37(1):870-878. doi: 10.1080/02656736.2020.1791365. PMID: 32689830.

Chen PS, Chiu WT, Hsu PL, Lin SC, Peng IC, Wang CY, Tsai SJ. Pathophysiological implications of hypoxia in human diseases. J Biomed Sci. 2020 May 11;27(1):63. doi: 10.1186/s12929-020-00658-7. PMID: 32389123; PMCID: PMC7212687.

Correia SC, Moreira PI. Hypoxia-inducible factor 1: a new hope to counteract neurodegeneration? J Neurochem. 2010 Jan; 112(1):1-12. doi: 10.1111/j.1471-4159.2009.06443.x. Epub 2009 Oct 20. PMID: 19845827.

Crawford JD, Terry ME, Rourke GM. Simplification of drug dosage calculation by application of the surface area principle. Pediatrics. 1950 May;5(5):783-90. PMID: 15417279.

Deepak V, Ramachandran S, Balahmar RM, Pandian SR, Sivasubramaniam SD, Nellaiah H, Sundar K. In vitro evaluation of anticancer properties of exopolysaccharides from Lactobacillus acidophilus in colon cancer cell lines. In Vitro Cell Dev Biol Anim. 2016 Feb;52(2):163-73. doi: 10.1007/s11626-015-9970-3. Epub 2015 Dec 10. PMID: 26659393.

Esfandiary A, Taherian-Esfahani Z, Abedin-Do A, Mirfakhraie R, Shirzad M, Ghafouri-Fard S, Motevaseli E. Lactobacilli Modulate Hypoxia-Inducible Factor (HIF)-1 Regulatory Pathway in Triple Negative Breast Cancer Cell Line. Cell J. 2016 Jul-Sep;18(2):237-244. doi: 10.22074/cellj.2016.4319. Epub 2016 May 30. PMID: 27540529; PMCID: PMC4988423.

Gibson PG, Qin L, Puah SH. COVID-19 acute respiratory distress syndrome (ARDS): clinical features and differences from typical pre-COVID-19 ARDS. Med J Aust. 2020 Jul;213(2):54-56.e1. doi: 10.5694/mja2.50674. Epub 2020 Jun 22. PMID: 32572965; PMCID: PMC7361309.

Gironi M, Bianchi A, Russo A, Alberoni M, Ceresa L, Angelini A, Cursano C, Mariani E, Nemni R, Kullmann C, Farina E, Martinelli Boneschi F. Oxidative imbalance in different neurodegenerative diseases with memory impairment. Neuro-degener Dis. 2011;8(3):129-37. doi: 10.1159/000319452. Epub 2010 Sep 13. PMID: 20838029.

Goda N, Kanai M. Hypoxia-inducible factors and their roles in energy metabolism. Int J Hematol. 2012 May;95(5):457-63. doi: 10.1007/s12185-012-1069-y. Epub 2012 Apr 26. PMID: 22535382.

Han DH, Kim WK, Park S, Jang YJ, Ko G. Lactobacillus paracasei treatment modulates mRNA expression in macrophages. Biochem Biophys Rep. 2020 Jul 21;23:100788. doi: 10.1016/j.bbrep. 2020.100788. PMID: 32715107; PMCID: PMC7374253.

Hu CJ, Wang LY, Chodosh LA, Keith B, Simon MC. Differential roles of hypoxia-inducible factor 1-alpha (HIF-1alpha) and HIF-2alpha in hypoxic gene regulation. Mol Cell Biol. 2003 Dec;23(24):9361-74. doi: 10.1128/mcb.23.24.9361-9374.2003. PMID: 14645546; PMCID: PMC309606.

Ivan M, Kondo K, Yang H, Kim W, Valiando J, Ohh M, Salic A, Asara JM, Lane WS, Kaelin WG Jr. HIFalpha targeted for VHL-mediated destruction by proline hydroxylation: implications for O2 sensing. Science. 2001 Apr 20;292(5516):464-8. doi: 10.1126/ science.1059817. Epub 2001 Apr 5. PMID: 11292862.

Karhausen J, Haase VH, Colgan SP. Inflammatory hypoxia: role of hypoxia-inducible factor. Cell Cycle. 2005 Feb;4(2):256-8. Epub 2005 Mar 1. PMID: 15655360.

Lee JW, Ko J, Ju C, Eltzschig HK. Hypoxia signaling in human diseases and therapeutic targets. Exp Mol Med. 2019 Jun 20;51(6):1-13. doi: 10.1038/s12276-019-0235-1. PMID: 31221962; PMCID: PMC6586801.

Legros A, Marshall HR, Beuter A, Gow J, Cheung B, Thomas AW, Prato FS, Stodilka RZ. Effects of acute hypoxia on postural and kinetic tremor. Eur J Appl Physiol. 2010 Sep; 110(1):109-19. doi: 10.1007/s00421-010-1475-x. Epub 2010 Apr 23. PMID: 20414673.

Lundquist P, Artursson P. Oral absorption of peptides and nanoparticles across the human intestine: Opportunities, limitations and studies in human tissues. Adv Drug Deliv Rev. 2016; 106(Pt B): 256-276.

Matheson PJ, Wilson MA, Garrison RN. Regulation of intestinal blood flow. J Surg Res. 2000 Sep;93(1):182-96. doi: 10.1006/jsre.2000.5862. PMID: 10945962.

McFarland LV, Evans CT, Goldstein EJC. Strain-Specificity and Disease-Specificity of Probiotic Efficacy: A Systematic Review and Meta-Analysis. Front Med (Lausanne). 2018 May 7;5:124. doi: 10.3389/fmed.2018.00124. PMID: 29868585; PMCID: PMC5949321.

Merelli A, Repetto M, Lazarowski A, Auzmendi J. Hypoxia, Oxidative Stress, and Inflammation: Three Faces of Neuro-degenerative Diseases. J Alzheimers Dis. 2020 Dec 2. doi: 10.3233/ JAD-201074. Epub ahead of print. PMID: 33325385.

Nalivaeva NN, Rybnikova EA. Editorial: Brain Hypoxia and Ischemia: New Insights Into Neurodegeneration and Neuro-protection. Front Neurosci. 2019 Jul 25;13:770. doi: 10.3389/fnins. 2019.00770. PMID: 31404249; PMCID: PMC6669960.

Oddo S, Caccamo A, Kitazawa M, Tseng BP, LaFerla FM. Amyloid deposition precedes tangle formation in a triple transgenic model of Alzheimer's disease. Neurobiol Aging. 2003 Dec;24(8):1063-70. doi: 10.1016/j.neurobiolaging.2003.08.012. PMID: 14643377.

Ogunshola OO, Antoniou X. Contribution of hypoxia to Alzheimer's disease: is HIF-1alpha a mediator of neuro-degeneration? Cell Mol Life Sci. 2009 Nov;66(22):3555-63. doi: 10.1007/s00018-009-0141-0. Epub 2009 Sep 11. PMID: 19763399.

Ramakrishnan SK, Shah YM. Role of Intestinal HIF-2α in Health and Disease. Annu Rev Physiol. 2016;78:301-25. doi: 10.1146/annurev-physiol-021115-105202. Epub 2015 Nov 19. PMID: 26667076; PMCID: PMC4809193.

Ramirez P, Gordón M, Martin-Cerezuela M, Villarreal E, Sancho E, Padrós M, Frasquet J, Leyva G, Molina I, Barrios M, Gimeno S, Castellanos A. Acute respiratory distress syndrome due to COVID-19. Clinical and prognostic features from a medical Critical Care Unit in Valencia, Spain. Med Intensiva. 2021 Jan-Feb;45(1):27-34. doi: 10.1016/j.medin.2020.06.015. Epub 2020 Jul 11. PMID: 32919796; PMCID: PMC7836701.

Rangel-Huerta E, Maldonado E. Transit-Amplifying Cells in the Fast Lane from Stem Cells towards Differentiation. Stem Cells Int. 2017;2017:7602951. doi: 10.1155/2017/7602951. Epub 2017 Aug 1. PMID: 28835754; PMCID: PMC5556613.

Schito L, Semenza GL. Hypoxia-Inducible Factors: Master Regulators of Cancer Progression. Trends Cancer. 2016 Dec;2(12): 758-770. doi: 10.1016/j.trecan.2016.10.016. Epub 2016 Nov 16. PMID: 28741521.

Shepherd AP. Metabolic control of intestinal oxygenation and blood flow. Fed Proc. 1982 Apr;41(6):2084-9. PMID: 7075783.

Singhal R, Shah YM. Oxygen battle in the gut: Hypoxia and hypoxia-inducible factors in metabolic and inflammatory responses in the intestine. J Biol Chem. 2020 Jul 24;295(30):10493-10505. doi: 10.1074/jbc.REV120.011188. Epub 2020 Jun 5. PMID: 32503843; PMCID: PMC7383395.

Sulser P, Pickel C, Günter J, Leissing TM, Crean D, Schofield CJ, Wenger RH, Scholz CC. HIF hydroxylase inhibitors decrease cellular oxygen consumption depending on their selectivity. FASEB J. 2020 Feb;34(2):2344-2358. doi: 10.1096/fj. 201902240R. Epub 2019 Dec 19. PMID: 31908020.

Toescu EC. Hypoxia response elements. Cell Calcium. 2004 Sep-Oct;36(3-4):181-5. doi: 10.1016/j.ceca.2004.02.020. PMID: 15261474.

Van Der Schoor SR, Reeds PJ, Stoll B, Henry JF, Rosenberger JR, Burrin DG, Van Goudoever JB. The high metabolic cost of a functional gut. Gastroenterology. 2002 Dec;123(6):1931-40. doi: 10.1053/gast.2002.37062. PMID: 12454850.

Wang GL, Jiang BH, Rue EA, Semenza GL. Hypoxia-inducible factor 1 is a basic-helix-loop-helix-PAS heterodimer regulated by cellular O2 tension. Proc Natl Acad Sci U S A. 1995 Jun 6;92(12):5510-4. doi: 10.1073/pnas.92.12.5510. PMID: 7539918; PMCID: PMC41725.

Wiener CM, Booth G, Semenza GL. In vivo expression of mRNAs encoding hypoxia-inducible factor 1. Biochem Biophys Res Commun. 1996 Aug 14;225(2):485-8. doi: 10.1006/bbrc.1996.1199. PMID: 8753788.

Wu D, Potluri N, Lu J, Kim Y, Rastinejad F. Structural integration in hypoxia-inducible factors. Nature. 2015 Aug 20;524(7565):303-8. doi: 10.1038/nature14883. Epub 2015 Aug 5. PMID: 26245371.

## Claims

1. Strain of *Lactobacillus acidophilus* deposited with the accession number CNCM I-5567 capable of increasing cellular levels of the hypoxia-inducible factor HIF-1α associated with the reduction of cellular oxygen consumption for use in the treatment of hypoxia-inducing conditions selected from the group consisting of chronic fatigue, oxygen deprivation, neurodegenerative diseases, neonatal hypoxia-ischemia, myocardial ischemia, metabolic disorders, chronic cardiac and renal diseases, reproductive disorders such as pre-eclampsia and endometriosis, exacerbation of postural and kinetic tremors, and cerebral hypoxia.

2. A composition comprising *Lactobacillus acidophilus* according to claim 1 and optionally one or more pharmaceutically acceptable excipients for use according to claim 1.

3. Composition according to claim 2 for use according to claim 1, further comprising the strain of *Streptococcus thermophilus* deposited with the access number CNCM I-5570, and the strain of *Bifidobacterium animalis subsp. lactis* deposited with the access number CNCM I-5571 and/or the strain of *Bifidobacterium animalis subsp. lactis* deposited with the access number CNCM I-5572.

4. Composition according to claim 2 or 3 for use according to claim 1, comprising from 30% to 50% by weight of *Lactobacillus acidophilus,* from 25% to 35% by weight of *Streptococcus thermophilus,* and from 25% to 35% by weight of *Bifidobacterium animalis subsp. lactis,* depending on the weight of the composition.

5. Composition according to any of claims 2 to 4 for use according to claim 1, further comprising the strain of *Levilactobacillus brevis* deposited with the accession number CNCM I-5566, the strain of *Lactiplantibacillus plantarum* subsp. *plantarum* deposited with the accession number CNCM I-5569, the strain of *Lacticaseibacillus paracasei* subsp. *paracasei* deposited with the accession number CNCM I-5568, and the strain of *Lactobacillus helveticus* deposited with the accession number CNCM I-5573.

6. Composition according to claim 5 for use according to claim 1, which comprises from 30% to 50% by weight *Lactobacillus acidophilus,* 1% to 10% by weight *Streptococcus thermophilus,* from 1% to 20% by weight *Bifidobacterium animalis* subsp. *lactis,* from 1% to 10% by weight *Levilactobacillus brevis* (formerly known as *Lactobacillus brevis),* from 1% to 10% by weight *Lactiplantibacillus plantarum* subsp. *plantarum* (formerly known as *Lactobacillus plantarum*), from 1% to 10% by weight of *Lacticaseibacillus paracasei* subsp. *paracasei* (formerly known as *Lactobacillus paracasei* subsp. *paracasei*), and from 1% to 10% by weight of *Lactobacillus helveticus,* based on the weight of the composition.

7. Composition according to any one of claims 2 to 6 for use according to claim 1, suitable for oral administration, such as in the form of powders, capsules or granules or sprays.

8. Composition according to claim 7 for use according to claim 1 having a high concentration of bacteria, to the extent of at least 10 billion in the adult and at least 100 million in the infant.

9. Composition for use according to claim 7, wherein said composition is suitable for administration to animals reared and/or maintained under oxygen-deficient conditions.

10. Probiotic bacterial strains or compositions according to any of the preceding claims for use according to claim 1, wherein the bacteria used are viable, non-viable, sonicated, tindalized or lyophilized.

## Patentansprüche

1. Stamm von *Lactobacillus acidophilus,* hinterlegt unter der Zugangsnummer CNCM I-5567, der zum Erhöhen zellulärer Spiegel des Hypoxie-induzierbaren Faktors HIF-1α imstande ist, der mit der Reduktion eines zellulären Sauerstoffverbrauchs in Verbindung steht, zur Verwendung bei der Behandlung von Hypoxie-induzierenden Bedingungen, die aus der Gruppe ausgewählt sind, bestehend aus chronischer Müdigkeit, Sauerstoffmangel, neurodegenerativen Erkrankungen, neonataler Hypoxie-Ischämie, Myokardischämie, Stoffwechselstörungen, chronischen Herz- und Nierenerkrankungen, Fortpflanzungsstörungen wie Präeklampsie und Endometriose, Verschlimmerung posturaler und kinetischer Tremores und zerebraler Hypoxie.

2. Zusammensetzung, umfassend *Lactobacillus acidophilus* nach Anspruch 1 und optional einen oder mehrere pharmazeutisch verträgliche Hilfsstoffe zur Verwendung nach Anspruch 1.

3. Zusammensetzung nach Anspruch 2 zur Verwendung nach Anspruch 1, ferner umfassend den Stamm von *Streptococcus thermophilus,* hinterlegt unter der Zugangsnummer CNCM I-5570, und den Stamm von *Bifidobacterium animalis subsp. lactis,* hinterlegt unter der Zugangsnummer CNCM I-5571, und/oder den Stamm von *Bifidobacterium animalis subsp. lactis,* hinterlegt unter der Zugangsnummer CNCM I-5572.

4. Zusammensetzung nach Anspruch 2 oder 3 zur Verwendung nach Anspruch 1, umfassend von zu 30 Gew.-% bis 50 Gew.-% *Lactobacillus acidophilus,* von zu 25 Gew.-% bis 35 Gew.-% *Streptococcus thermophilus* und von zu 25 Gew.-% bis 35 Gew.-% *Bifidobacterium animalis subsp. lactis,* abhängig von dem Gewicht der Zusammensetzung.

5. Zusammensetzung nach einem der Ansprüche 2 bis 4 zur Verwendung nach Anspruch 1, ferner umfassend den Stamm von *Levilactobacillus brevis,* hinterlegt unter der Zugangsnummer CNCM I-5566, den Stamm von *Lactiplantibacillus plantarum* Subsp. *plantarum,* hinterlegt unter der Zugangsnummer CNCM I-5569, den Stamm von *Lacticaseibacillus paracasei* Subsp. *paracasei,* hinterlegt unter der Zugangsnummer CNCM I-5568, und den Stamm von *Lactobacillus helveticus,* hinterlegt unter der Zugangsnummer CNCM I-5573.

6. Zusammensetzung nach Anspruch 5 zur Verwendung nach Anspruch 1, umfassend von zu 30 Gew.-% bis 50 Gew.-% *Lactobacillus acidophilus,* zu 1 Gew.-% bis 10 Gew.-% *Streptococcus thermophilus,* von zu 1 Gew.-% bis 20 Gew.-% *Bifidobacterium animalis* Subsp. *lactis,* von zu 1 Gew.-% bis 10 Gew.-% *Levilactobacillus brevis* (vormals bekannt als *Lactobacillus brevis),* von zu 1 Gew.-% bis 10 Gew.-% *Lactiplantibacillus plantarum* Subsp. *plantarum* (vormals bekannt als *Lactobacillus plantarum*), von zu 1 Gew.-% bis 10 Gew.-% *Lacticaseibacillus paracasei* Subsp. *paracasei* (vormals bekannt als *Lactobacillus paracasei* Subsp. *paracasei*), und von zu 1 Gew.-% bis 10 Gew.-% *Lactobacillus helveticus,* basierend auf dem Gewicht der Zusammensetzung.

7. Zusammensetzung nach einem der Ansprüche 2 bis 6 zur Verwendung nach Anspruch 1, die für eine orale Verabreichung, wie in der Form von Pulvern, Kapseln oder Granulaten oder Sprays, geeignet ist.

8. Zusammensetzung nach Anspruch 7 zur Verwendung nach Anspruch 1, die eine hohe Konzentration an Bakterien, in dem Umfang von mindestens 10 Milliarden bei dem Erwachsenen und mindestens 100 Millionen bei dem Säugling, aufweist.

9. Zusammensetzung zur Verwendung nach Anspruch 7, wobei die Zusammensetzung zur Verabreichung an Tiere geeignet ist, die unter sauerstoffarmen Bedingungen aufgezogen und/oder gehalten werden.

10. Probiotische Bakterienstämme oder Zusammensetzungen nach einem der vorstehenden Ansprüche zur Verwendung nach Anspruch 1, wobei die verwendeten Bakterien lebensfähig, nicht lebensfähig, beschallt, tyndallisiert oder lyophilisiert sind.

## Revendications

1. Souche de *Lactobacillus acidophilus* déposée sous le numéro d'accès CNCM I-5567 capable d'une augmentation des niveaux cellulaires du facteur inductible par l'hypoxie HIF-1α associée à la réduction de la consommation cellulaire d'oxygène, pour une utilisation dans le traitement d'affections induisant l'hypoxie, choisies dans le groupe constitué de fatigue chronique, privation d'oxygène, maladies neurodégénératives, hypoxie-ischémie néonatale, ischémie myocardique, troubles métaboliques, maladies cardiaques et rénales chroniques, troubles de la reproduction tels que la prééclampsie et l'endométriose, exacerbation des tremblements posturaux et cinétiques et hypoxie cérébrale.

2. Composition comprenant *Lactobacillus acidophilus* selon la revendication 1 et éventuellement un ou plusieurs excipients pharmaceutiquement acceptables pour une utilisation selon la revendication 1.

3. Composition selon la revendication 2 pour une utilisation selon la revendication 1, comprenant en outre la souche de *Streptococcus thermophilus* déposée sous le numéro d'accès CNCM I-5570 et la souche de *Bifidobacterium animalis subsp. lactis* déposée sous le numéro d'accès CNCM I-5571 et/ou la souche de *Bifidobacterium animalis subsp. lactis* déposée sous le numéro d'accès CNCM I-5572.

4. Composition selon la revendication 2 ou 3 pour une utilisation selon la revendication 1, comprenant 30% à 50% en poids de *Lactobacillus acidophilus,* 25% à 35% en poids de *Streptococcus thermophilus* et 25% à 35% en poids de *Bifidobacterium animalis subsp. lactis,* en fonction du poids de la composition.

5. Composition selon l'une quelconque des revendications 2 à 4 pour une utilisation selon la revendication 1, comprenant en outre la souche de *Levilactobacillus brevis* déposée sous le numéro d'accès CNCM I-5566, la souche de *Lactiplantibacillus plantarum* subsp. *plantarum* déposée sous le numéro d'accès CNCM I-5569, la souche de *Lacticaseibacillus paracasei* subsp. *paracasei* déposée sous le numéro d'accès CNCM I-5568 et la souche de *Lactobacillus helveticus* déposée sous le numéro d'accès CNCM I-5573.

6. Composition selon la revendication 5 pour une utilisation selon la revendication 1, qui comprend 30% à 50% en poids de *Lactobacillus acidophilus,* 1% à 10% en poids de *Streptococcus thermophilus,* 1% à 20% en poids de *Bifidobacterium animalis* subsp. *lactis,* 1% à 10% en poids de *Levilactobacillus brevis* (précédemment connu sous le nom de *Lactobacillus brevis),* 1% à 10% en poids de *Lactiplantibacillus plantarum* subsp. *plantarum* (précédemment connu sous le nom de *Lactobacillus plantarum*), 1% à 10% en poids de *Lacticaseibacillus paracasei* subsp. *paracasei* (précédemment connu sous le nom de *Lactobacillus paracasei* subsp. *paracasei)* et 1% à 10% en poids de *Lactobacillus helveticus,* sur la base du poids de la composition.

7. Composition selon l'une quelconque des revendications 2 à 6 pour une utilisation selon la revendication 1, appropriée pour une administration par voie orale, telle que sous forme de poudres, de capsules ou de granulés ou de sprays.

8. Composition selon la revendication 7 pour une utilisation selon la revendication 1 ayant une concentration élevée en bactéries, à hauteur d'au moins 10 milliards chez l'adulte et d'au moins 100 millions chez le nourrisson.

9. Composition pour une utilisation selon la revendication 7, dans laquelle ladite composition est appropriée pour une administration à des animaux élevés et/ou maintenus dans des conditions déficientes en oxygène.

10. Souches bactériennes probiotiques ou compositions selon l'une quelconque des revendications précédentes pour une utilisation selon la revendication 1, dans lesquelles les bactéries utilisées sont viables, non viables, soniquées, tyndallisées ou lyophilisées.
